# EUROPEAN PATENT APPLICATION

(11) **EP 3 888 555 A1**
(43) Date of publication of application: **06.10.2021**
(21) Application number: 19889594.8
(22) Date of filing: 19.02.2019
(51) Int. Cl.: A61B 8/00, A61B 8/08

(54) **TRANSMITTING/RECEIVING DUAL-MODE FOCUSED ULTRASONIC TRANSDUCER AND MICROBUBBLE CAVITATION IMAGE VISUALIZATION METHOD USING SAME**

(30) Priority: 28.11.2018 KR 20180149145
(71) Applicant: Daegu-Gyeongbuk Medical Innovation Foundation, Daegu 41061 (KR)
(72) Inventor: PARK, Juyoung, Gyeongsan-si, Gyeongsangbuk-do 38538 (KR); JIN, Chang Zhu, Daegu 41066 (KR); HUH, Hyung Kyu, Daegu 41061 (KR)
(74) Representative: EP&C
(86) International application number: PCT/KR2019/001968
(87) International publication number: WO 2020/111390

(57) **Abstract**

The present invention relates to a transmitting/receiving dual-mode focused ultrasonic transducer and a microbubble cavitation image visualization method using the transducer. More particularly, the present invention relates to a transmitting/receiving dual-mode focused ultrasonic transducer and a microbubble cavitation image visualization method using the transducer, wherein a plurality of mounting holes are formed in a transducer body with a limited area according to the Fibonacci pattern that allows for the maximum number of objects to be mounted in the transducer body, and a plurality of transducer elements is mounted in the mounting holes so as to form a transducer element arrangement having highly nonlinearity, whereby microbubble cavitation can be induced and visualized by using a small number of receiving elements.

## Description

### Technical Field

The present invention relates to a transmitting/receiving dual-mode focused ultrasonic transducer and a microbubble cavitation image visualization method using the transducer. More particularly, the present invention relates to a transmitting/receiving dual-mode focused ultrasonic transducer and a microbubble cavitation image visualization method using the transducer, wherein a plurality of mounting holes are formed in a transducer body with a limited area according to the Fibonacci pattern that allows for the maximum number of objects to be mounted in the transducer body, and a plurality of transducer elements is mounted in the mounting holes so as to form a transducer element arrangement having highly nonlinearity, whereby microbubble cavitation can be induced and visualized by using a small number of receiving elements.

### Background Art

A technology for controlling blood brain barrier opening and closure using focused ultrasound is a new non-invasive brain cancer/brain tumor treatment technology, which temporarily opens the blood brain barrier (BBB) in a safe and local manner to accurately deliver the therapeutic drug to the target location. According to the technology, the blood brain barrier is physically opened by irradiating the affected area with focused ultrasound after intravenous injection of an ultrasound contrast agent (microbubbles) that is commonly used in ultrasound imaging and thus inducing the movement (hereinafter, referred to as cavitation) of the microbubbles. Accordingly, there is a need to develop a technology to visualize and monitor the generated cavitation signal in a 3D manner. Although only equipment manufactured by Company I in clinical use has an ultrasonic transducer consisting of hundreds of independent elements, a cavitation visualization function has not been developed yet.

The cavitation signals generated by microbubbles during treatment cannot be detected using imaging equipment such as MRI and CT in the related art, and only ultrasonic transducers capable of sound wave detection can measure such signals. Accordingly, there is a need to develop a dual-mode focused ultrasonic transducer, which is capable of reducing the number of elements used in therapeutic ultrasonic transducers to a minimum and detecting cavitation signals by microbubbles.

Korean Patent Publication No. 2016-0023276 (hereinafter, referred to as a literature in the related art) discloses a method and apparatus for generating high intensity focused ultrasound, in which transducers with different resonant frequencies are combined to have a plurality of resonant frequencies and treatment depths and high-intensity focused ultrasound waves are emitted from the handpiece to the treatment site.

However, the literature in the related art discloses only an arrangement of a transducer having a plurality of resonant frequencies, and does not disclose efficient arrangement of a plurality of transducer elements in a large amount that makes it possible to increase nonlinearity and to improve the quality of an ultrasound image.

### Disclosure

### Technical Problem

Accordingly, the present invention has been made keeping in mind the above problems occurring in the related art, and an objective of the present invention is to provide a transmitting/receiving dual-mode focused ultrasonic transducer, which is capable of mounting the maximum number of elements in a limited area by implementing a pattern of a transducer with high nonlinearity.

In addition, another objective of the present invention is to provide a microbubble cavitation image visualization method, which is capable of reducing the formation of virtual images and improving the quality of an ultrasound image.

### Technical Solution

In order to achieve the above objectives, a transmitting/receiving dual-mode focused ultrasonic transducer according to an embodiment of the present invention includes a transducer body having a concave curved shape and having a plurality of mounting holes formed in a Fibonacci pattern; and a plurality of transducer elements configured to be detachably mounted in the plurality of mounting holes, respectively, to transmit and receive ultrasonic waves.

In the transmitting/receiving dual-mode focused ultrasonic transducer according to an embodiment of the present invention, the transducer element may be configured so that a transmitter and a receiver are arranged in a coaxial shape.

In the transmitting/receiving dual-mode focused ultrasonic transducer according to an embodiment of the present invention, the transmitter may be formed in a cylindrical shape having a ring shape when viewed from the top.

In the transmitting/receiving dual-mode focused ultrasonic transducer according to an embodiment of the present invention, the receiver may be configured to be mounted on an inner circumference of the transmitter.

In the transmitting/receiving dual-mode focused ultrasonic transducer according to an embodiment of the present invention, the transmitter and the receiver may be made of piezoelectric elements having different resonant frequencies from each other.

A microbubble cavitation image visualization method using the transmitting/receiving dual-mode focused ultrasonic according to another embodiment of the present invention includes inputting an external trigger signal to a transducer; transmitting, by a transmitter, a sine wave signal having a frequency f₀ in the form of a tone burst to microbubbles for a predetermined time; receiving, by a receiver, a signal reflected from the microbubbles; calculating an image frame by transmitting the reflected signal to a beamforming unit; and collecting the image frame calculated by a video stack construction unit, to configure one video stack.

### Advantageous Effects

A transmitting/receiving dual-mode focused ultrasonic transducer according to an embodiment of the present invention includes a transducer body having a concave curved shape and having a plurality of mounting holes formed in a Fibonacci pattern; and a plurality of transducer elements configured to be detachably mounted in the plurality of mounting holes, respectively, to transmit and receive ultrasonic waves, whereby it is possible to implement the transducer pattern with high nonlinearity while mounting the maximum number of transducer elements in the transducer body with a limited area, and thus to improve the quality of an ultrasound image and effectively visualize the location of microbubbles in a 3D space through signals received using a transmission/reception module.

In addition, a microbubble cavitation image visualization method using the transmitting/receiving dual-mode focused ultrasonic according to an embodiment of the present invention, the method including inputting an external trigger signal to a transducer; transmitting, by a transmitter, a sine wave signal having a frequency f₀ in the form of a tone burst to microbubbles for a predetermined time; receiving, by a receiver, a signal reflected from the microbubbles; calculating an image frame by transmitting the reflected signal to a beamforming unit; and collecting the image frame calculated by a video stack construction unit, to configure one video stack, whereby there is an excellent effect that it is possible to visualize high quality cavitation images of microbubbles with reduced virtual image formation.

### Description of Drawings

FIG. 1 is a block diagram of a transmitting/receiving dual-mode focused ultrasonic transducer according to an embodiment of the present invention.
FIG. 2 is a diagram illustrating a Fibonacci pattern applied to a transducer body of FIG. 1, in which (a) is a diagram illustrating the Fibonacci pattern that allows for the maximum number of objects to be mounted in a limited area, and (b) is a diagram illustrating an arrangement of transducer elements based on a Fibonacci pattern.
FIG. 3 is a diagram illustrating the Fibonacci pattern of mounting holes of the transducer body of FIG. 1, in which (a) is a diagram illustrating an arrangement of eight mounting holes, (b) is a diagram illustrating an arrangement of 40 mounting holes, and (c) is a diagram illustrating an arrangement of 64 mounting holes.
FIG. 4 is a diagram illustrating the structure and operating principle of each element of the transducer of FIG. 1, indicating that transducer elements (piezoelectric elements) having different resonant frequencies in a transmitter and a receiver are mounted, and the transmitter and the receiver are used independently to reduce the influence of the receiver on the transmission frequency, and ultrasonic waves generation and microbubble cavitation signal collection are capable of being simultaneously operated.
FIG. 5 is a diagram illustrating that when a trigger signal is input to the transducer element of FIG. 1, a signal is transmitted from a transmitter and a signal reflected from microbubbles is received by a receiver and then transmitted to a beamforming unit to calculate an image frame.
FIG. 6 (a) is a diagram illustrating sound visualization in a space using the receiver of the transducer element of FIG. 1, and FIG. 6 (b) is a diagram illustrating an image frame reconstructed using the signal received by the receiver of the transducer element.
FIG 7 is a flowchart illustrating a microbubble cavitation image visualization method using a transducer according to an embodiment of the present invention.

### Mode for Invention

Hereinafter, embodiments of the present invention will be described in detail with reference to the drawings.

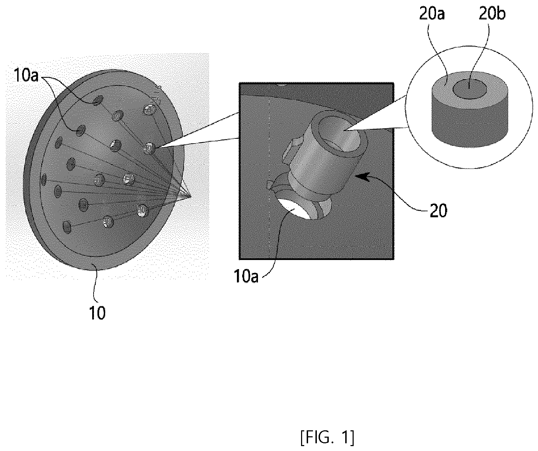

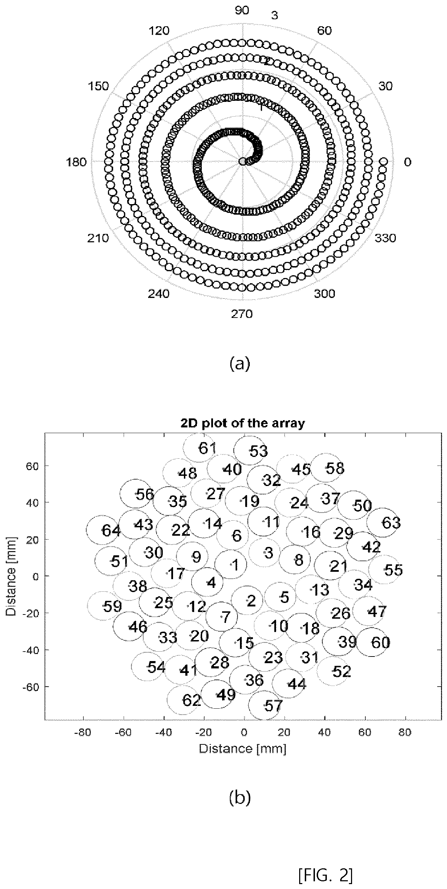

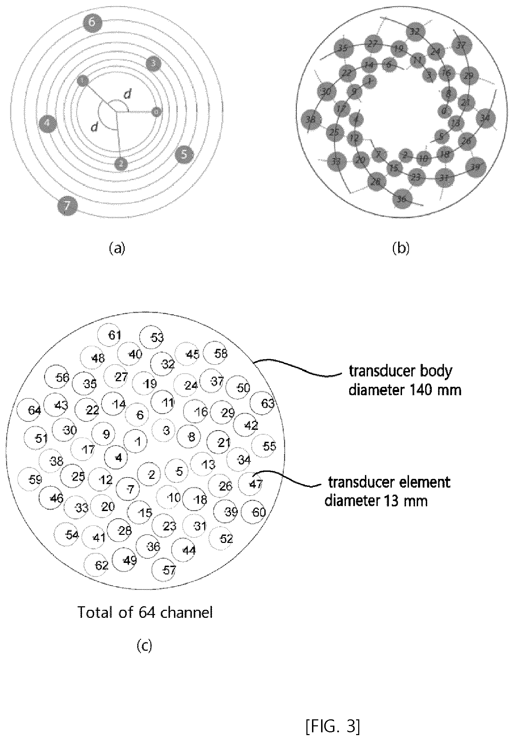

FIG. 1 is a block diagram of a transmitting/receiving dual-mode focused ultrasonic transducer according to an embodiment of the present invention; FIG. 2 is a diagram illustrating a Fibonacci pattern applied to a transducer body of FIG. 1, in which (a) is a diagram illustrating the Fibonacci pattern that allows for the maximum number of objects to be mounted in a limited area, and (b) is a diagram illustrating an arrangement of transducer elements based on a Fibonacci pattern; and FIG. 3 is a diagram illustrating the Fibonacci pattern of mounting holes of the transducer body of FIG. 1, in which (a) is a diagram illustrating an arrangement of eight mounting holes, (b) is a diagram illustrating an arrangement of 40 mounting holes, and (c) is a diagram illustrating an arrangement of 64 mounting holes.

A transmitting/receiving dual-mode focused ultrasonic transducer according to an embodiment of the present invention includes a transducer body 10 and a transducer element 20, as shown in FIGS. 1 to 3.

The transducer body 10 is configured in a concave curved shape to which a plurality of transducer elements 20 is fixed so that ultrasonic waves may be focused at one point, and has a plurality of mounting holes 10a formed in a Fibonacci pattern that allows increasing nonlinearity while mounting the maximum number of transducer elements 20 within a limited area.

The Fibonacci pattern is an optimal pattern that is capable of mounting the largest number of objects in a small area, as a pattern that exists universally in nature. FIG. 2 (a) is a diagram illustrating a Fibonacci pattern that allows for the maximum number of objects to be mounted in a limited area. FIG. 2 (b) is a diagram illustrating an arrangement of transducer elements based on the Fibonacci pattern, indicating that transducer elements in the maximum number are arranged in the Fibonacci pattern in a space with limited horizontal and vertical lengths.

FIG. 3 is a diagram illustrating an arrangement in which mounting holes 10a of the transducer body of FIG. 1 are formed in the Fibonacci pattern, in which (a) is a diagram illustrating an arrangement of eight mounting holes, (b) is a diagram illustrating an arrangement of 40 mounting holes, and (c) is a diagram illustrating an arrangement of 64 mounting holes. The number of transducer elements mounted in the mounting holes 10a represents the number of channels.

Since the transducer elements 20 are arranged in a nonlinear pattern manner, the formation of virtual images may be reduced compared to when using a linear array, and the number of elements capable of being mounted in a limited area may be increased and the reduction of the volume of the entire transducer may be reduced compared to the related art. Accordingly, the total weight of the manufactured transducer may be reduced, and thus the freedom degree of movement of the transducer including the transducer body may be increased.

The transducer element 20 is mounted in each of the plurality of mounting holes 10a of the transducer body 10 and serves to transmit and receive ultrasonic waves. It is possible to configure the Fibonacci pattern more elaborately by manufacturing the transducer body 10 in the Fibonacci pattern quantified in 3D modeling using 3D printing technology. In addition, each transducer element 20 is configured in such a manner as to be detachable from the transducer body 10, so that when some elements need replacement due to aging, only the corresponding elements may be replaced. Rather than the manufacturing method of attaching each transducer element directly to the transducer body in the related art, the transducer element according to the present invention may be independently manufactured, so that it is possible to mass-produce the transducer elements with a specific performance and to evaluate the acoustic output performance of the transducer element before assembling the transducer, thereby maximizing the efficiency of the manufacturing process.

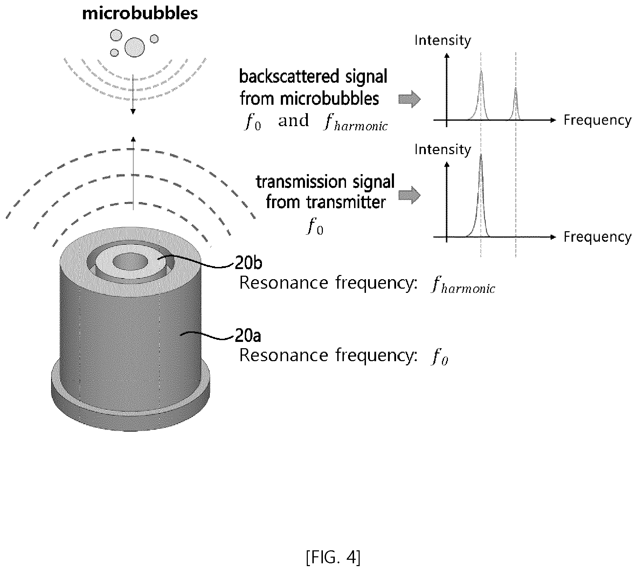

As shown in FIG. 4, the transducer element 20 is configured so that a transmitter 20a for transmitting ultrasonic waves toward the microbubbles and a receiver 20b for receiving signals reflected from the microbubbles are arranged in a coaxial shape, respectively, thereby increasing the number of transducer elements 20 capable of being mounted on a limited area. In addition, since the receiver 20b receives only the harmonic signal 2f₀ with respect to the transmission signal f₀, it is possible to fundamentally block the interference between the transmission/reception signals.

According to an embodiment, the transmitter 20a is formed in a cylindrical shape having a ring shape when viewed from the top, and the receiver 20b is formed in a cylindrical shape so as to be mounted inside the transmitter 20a. The transmitter 20a and the receiver 20b are combined to form the transducer element 20, which is mounted in the mounting hole 10a, thereby further increasing the number of transducer elements 20 capable of being mounted in a limited area, compared to when the transmitter and the receiver are each mounted in separate mounting holes.

The receiver 20b and the transmitter 20a physically use different piezoelectric elements from each other, and thus are connected to different electrical systems to implement simultaneous operation. This makes it possible to simultaneously induce microbubble cavitation while monitoring microbubble cavitation.

In addition, as shown in FIG. 4, the transducer element 20 may be configured so that the transmitter 20a and the receiver 20b are made of piezoelectric elements having different resonant frequencies from each other, and the influence on the receiver 20b by the transmission frequency is reduced by using the transmitter 20a and the receiver 20b independently, whereby it is possible to generate ultrasonic waves (induce cavitation of microbubbles) and collect and monitor cavitation signals from microbubbles.

Hereinafter, the operation of the transmitting/receiving dual-mode focused ultrasonic transducer according to an embodiment of the present invention configured as described above will be described.

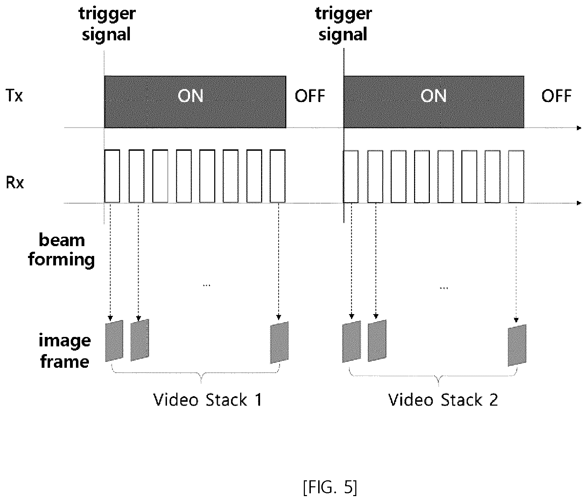

FIG. 5 is a diagram illustrating that when a trigger signal is input to the transducer element of FIG. 1, a signal is transmitted from a transmitter and a signal reflected from microbubbles is received by a receiver and then transmitted to a beamforming unit to calculate an image frame.

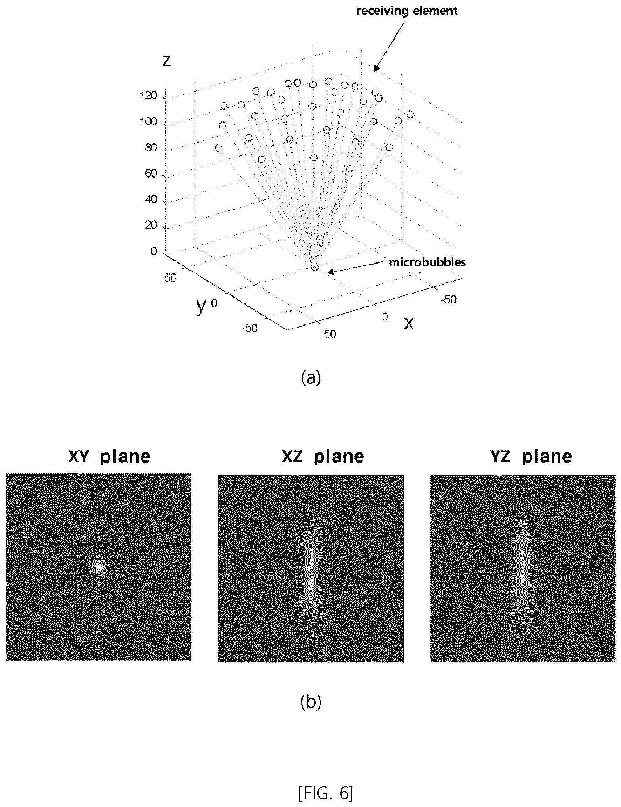

FIG. 6 (a) is a diagram illustrating sound visualization in a space using the receiver of the transducer element of FIG. 1, and FIG. 6 (b) is a diagram illustrating an image frame reconstructed using the signal received by the receiver of the transducer element.

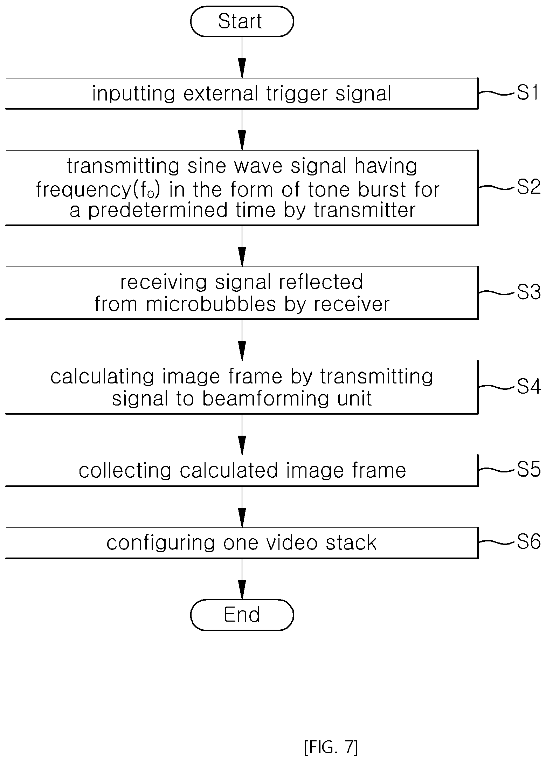

FIG 7 is a flowchart illustrating a microbubble cavitation image visualization method using a transducer according to an embodiment of the present invention.

First, when an external trigger signal is input to the transducer (S1), a sine wave signal having a frequency f₀ in the form of a tone burst is transmitted to the microbubbles for a time set by a transmitter 20a (S2), and signals (frequency nf₀, n = 2, 3, 4, etc.) reflected from the micro-bubbles are received by the receiver 20b by a certain sample (S3), and then transmitted to a beamforming unit (not shown) to calculate an image frame (S4).

Then, the calculated image frame is collected by the video stack configuration unit (not shown) (S5), to configure one video stack (S6).

Meanwhile, the one video stack may be used to immediately play an image through a display device such as a monitor or stored in a memory to prepare for post-processing use. The above operation is repeated according to the number of trigger signals input from the outside.

Meanwhile, in a step S4, the signals received by the plurality of receivers 20b are processed through a time exposure acoustic beamforming technique to visualize the location of an acoustic source, that is, microbubbles in a three-dimensional space, which is as shown in (a) of FIG. 6.

Using the signals received from the receiving element, that is, the plurality of receivers 20b, the image frame reconstructed by visualizing the cavitation image of microbubbles is divided into 2D regions for each of xy, yz, and zx planes to be displayed, which is as shown in FIG. 6 (b).

The cavitation signal generated by microbubbles cannot be detected with the existing imaging equipment such as MRI and CT, and can only be detected with an ultrasonic transducer capable of sound wave detection. However, equipment in the related art did not have the function of visualizing cavitation of microbubbles. Meanwhile, according to the present invention, it becomes possible to visualize and monitor the cavitation signal of microbubbles in a three-dimensional manner, as shown in FIG. 6.

A transmitting/receiving dual-mode focused ultrasonic transducer according to an embodiment of the present invention includes a transducer body having a concave curved shape and having a plurality of mounting holes formed in a Fibonacci pattern; and a plurality of transducer elements configured to be detachably mounted in the plurality of mounting holes, respectively, to transmit and receive ultrasonic waves, whereby it is possible to implement the transducer pattern with high nonlinearity while mounting the maximum number of transducer elements in the transducer body with a limited area, and thus to improve the quality of an ultrasound image and effectively visualize the location of microbubbles in a 3D space through signals received using a transmission/reception module.

In addition, a microbubble cavitation image visualization method using the transmitting/receiving dual-mode focused ultrasonic according to an embodiment of the present invention, the method including inputting an external trigger signal to a transducer; transmitting, by a transmitter, a sine wave signal having a frequency f₀ in the form of a tone burst to microbubbles for a predetermined time; receiving, by a receiver, a signal reflected from the microbubbles; calculating an image frame by transmitting the reflected signal to a beamforming unit; and collecting the image frame calculated by a video stack construction unit, to configure one video stack, whereby there is an excellent effect that it is possible to visualize high quality cavitation images of microbubbles with reduced virtual image formation.

In the drawings and specification, although an optimal embodiment has been disclosed, and specific terms have been used, this is used only for the purpose of describing the embodiments of the present invention, and is not used to limit the meaning or the scope of the present invention described in the claims. Therefore, those of ordinary skill in the art will understand that various modifications and equivalent other embodiments are possible therefrom. Therefore, the true technical scope of the present invention should be determined by the technical spirit of the appended claims.

## Claims

1. A transmitting/receiving dual-mode focused ultrasonic transducer, comprising:
a transducer body (10) having a concave curved shape and having a plurality of mounting holes (10a) formed in a Fibonacci pattern; and
a plurality of transducer elements (20) configured to be detachably mounted in the plurality of mounting holes, respectively, to transmit and receive ultrasonic waves.

2. The transducer of claim 1, wherein the transducer element (20) is configured so that a transmitter (20a) and a receiver (20b) are arranged in a coaxial shape.

3. The transducer of claim 2, wherein the transmitter (20a) is formed in a cylindrical shape having a ring shape when viewed from the top, and the receiver (20b) is configured to be mounted on an inner circumference of the transmitter.

4. The transducer of claim 2, wherein the transmitter (20a) and the receiver (20b) are made of piezoelectric elements having different resonant frequencies from each other.

5. A microbubble cavitation image visualization method using the transmitting/receiving dual-mode focused ultrasonic of claim 1, the method comprising:
inputting an external trigger signal to a transducer (S1);
transmitting, by a transmitter, a sine wave signal having a frequency fo in the form of a tone burst to microbubbles for a predetermined time (S2);
receiving, by a receiver (20b), a signal reflected from the microbubbles (S3);
calculating an image frame by transmitting the reflected signal to a beamforming unit (S4); and
collecting the image frame calculated by a video stack construction unit (S5), to configure one video stack (S6).
